# EUROPÄISCHE PATENTSCHRIFT

(11) **EP 1 100 477 B1**
(45) Veröffentlichungstag und Bekanntmachung des Hinweises auf die Patenterteilung: **14.04.2004**
(21) Anmeldenummer: 99936555.4
(22) Anmeldetag: 16.07.1999
(51) Int. Cl.: A61K 9/70

(54) **Pflaster, die zur transdermalen Applikation von Estradiol geeignet sind, und Wollwachs oder Bestandteile davon sowie Zinkoxid enthalten**
Patch comprising wool fat or its components and zinc oxide, suitable for the transdermal administration of estradiol
Timbre comprenant du cire de laine ou ses components et de l'oxyde de zinc, destiné a l'administration transdermique de l'estradiol

(30) Priorität: 29.07.1998 DE 19834007
(43) Veröffentlichungstag der Anmeldung: 23.05.2001
(73) Patentinhaber: LTS Lohmann Therapie-Systeme AG, 56626 Andernach (DE)
(72) Erfinder: KIRSTGEN, Elvira, D-56564 Neuwied (DE); MECONI, Reinhold, D-56567 Neuwied (DE)
(74) Vertreter: Flaccus, Rolf-Dieter, Dr.
(86) Internationale Anmeldenummer: PCT/EP1999/005085
(87) Internationale Veröffentlichungsnummer: WO 2000/006131

(56) Entgegenhaltungen:
- WO-A-96/19976
- DE-C- 4 416 927

## Beschreibung

Die Erfindung betrifft ein wirkstoffhaltiges Pflaster zur kontrollierten Abgabe von Estradiol oder seinen pharmazeutisch unbedenklichen Derivaten allein oder in Kombination mit Gestagenen an die menschliche oder tierische Haut.

Oestrogen- und/oder gestagenhaltige Pflaster sind bereits bekannt. Sie weisen jedoch die Nachteile auf, daß sie entweder Ethanol enthalten oder die potentielle Gefahr bergen, daß der Wirkstoff im Laufe der Zeit rekristallisiert.

Aus der DE-OS 32 05 258 und der EP 0 285 563 ist bekannt, Estradiol und Ethanol gleichzeitig in einer Pflasterformulierung zu verabreichen. Die Herstellung dieses Pflasters ist jedoch sehr aufwendig und der Tragekomfort nach Applikation aufgrund der fehlenden Flexibilität gering.

Die EP 0 285 563 beschreibt ein transdermales therapeutisches System für die kombinierte Applikation von Oestrogenen und Gestagenen. Das Reservoir erhält die Wirkstoffformulierung und gegebenenfalls eine Membran sowie Ethanol als perkutanes absorptionsverbesserndes Mittel. Da die Freisetzung des Wirkstoffs hauptsächlich von der Membran gesteuert wird, unterscheidet sich dieses transdermale therapeutische System grundsätzlich von dem Wirkstoffpflaster gemäß der vorliegenden Erfindung. Der Kleber hat bei dem dort beschriebenen Pflaster lediglich die Funktion, das Pflaster auf der Haut zu befestigen. Daß er einen Beitrag zur Steuerung der Wirkstofffreisetzung zu leisten vermag, ist nicht seine Hauptaufgabe, sondern lediglich ein - möglicherweise sogar unerwünschter - Nebeneffekt. Es handelt sich hierbei um ein sogenanntes "Beutelpflaster", da sich die Wirkstoffzubereitung in einem Beutel, bestehend aus undurchlässiger Rückschicht und Membran mit Kleberschicht befindet. Infolge seines komplizierten Aufbaues ist die Herstellung des Pflasters sehr aufwendig, da die Einzelkomponenten separat hergestellt und dann in einem weiteren Arbeitsgang zu einem Pflaster zusammengefügt werden müssen.

Die EP 0 275 716 beschreibt ein - im Gegensatz zu dem erfindugsgemäßen einschichtigen System - zweischichtiges transdermales System zur simultanen Verabreichung von einem oder mehreren Oestrogenen, die in der Polymerschicht gelöst oder mikrodispergiert sind. Die Haftschicht enthält dabei außer den Wirkstoffen Substanzen, die die transdermale Absorption verbessern. Polymer- und Haftschichten können aus Polyaxrylaten, Silikonen oder Polyisobutylenen bestehen.

Die EP 0 328 806 beschreibt ein membranfreies transdermales therapeutisches System, dessen Matrix aus einem Polyacrylatkleber, einem Lösemittel, einem Penetrationsverbesserer und Oestrogenen, dessen Derivaten und Kombinationen davon besteht.

In der WO 87/07138 ist ein Estradiol-Pflaster mit einer Rückschicht, einer den Wirkstoff enthaltenden Matrix und einem Haftkleber, der mit einer wiederablösbaren Schutzschicht abgedeckt ist, beschrieben. Die Herstellung von Matrix und Haftkleber erfolgen in technologisch sehr aufwendigen Arbeitsvorgängen durch Homogenisieren, Entgasen, Beschichten, Trocknen und Vereinzeln. In einer Ausführungsform muß die Rückschicht sogar mit einem Haftkleber beschichtet werde, was einen weiteren Arbeitsgang bedingt. Das Zusammenfügen der einzelnen Teile erfolgt in einem separaten Arbeitsgang. Die Herstellung des Pflasters ist also insgesamt sehr aufwendig und kompliziert.

Aus der US 4 624 665 sind Systeme bekannt, die im Reservoir den Wirkstoff in mikroverkapselter Form enthalten. Das Reservoir ist eingebettet zwischen Rückschicht und einer Membran. Der äußere Rand des Systems ist mit einem Haftkleber ausgerüstet. Der Aufbau und die Herstellung dieses Systems ist sehr kompliziert, da der Wirkstoff mikroverkapselt und in einer flüssigen Phase homogen verteilt werden muß, die dann in weiteren Arbeitsgängen zwischen Rückschicht und Membran eingebettet wird. Zusätzlich muß das System dann mit einem klebenden Rand versehen und mit einer Schutzschicht abgedeckt werden.

Es sind weiterhin aus der EP 0 186 019 Wirkstoffpflaster bekannt, bei denen einer Kautschuk/Klebharzmasse in Wasser quellbare Polymere zugesetzt sind und aus denen Estradiol freigesetzt werden kann. Es hat sich jedoch gezeigt, daß die Freisetzung von Estradiol aus diesen Wirkstoffpflastern viel zu gering ist und nicht den therapeutischen Erfordernissen entspricht.

In der DE-OS 20 06 969 ist ein Pflaster oder ein Haftverband mit Systemwirkung beschrieben, bei dem empfängnisverhütende Substanzen in der Klebstoffkomponente oder dem Klebstoffilm eingearbeitet sind. Der Klebstoffilm kann ein Acrylat sein.

Es ist somit Aufgabe der vorliegenden Erfindung; die oben aufgeführten Nachteile zu vermeiden und ein stabiles, das heißt rekristallisationsfreies oestrogen- und/oder gestagenhaltiges Pflaster mit ausreichender Wirkstofffreisetzung zur Verfügung zu stellen, dessen Freisetzung sich durch Lagerung nicht verändert.

Überraschend hat sich gezeigt, daß die Aufgabe durch einen oestrogen- und/oder gestagenhaltigen Haftkleber, der Wollwachs oder Bestandteile davon und Zinkoxid enthält, gelöst wird.

Die oben gestellte Aufgabe wird daher durch ein Wirkstoffhaltiges Pflaster gemäß Hauptanspruch gelöst. Die Unteransprüche betreffen besonders bevorzugte Ausführungsformen des Erfindungsgegenstandes.

Wollwachs ist als pharmazeutischer Rohstoff bekannt und als solcher in den Pharmacopoen beschrieben. Es wird verwendet aufgrund seines Liberationsvermögens für Wirkstoffe, seiner Hautfreundlichkeit und seines Wasseraufnahmevermögens.

Auch Zinkoxid ist in den Pharmacopoen beschrieben. Zinkoxid wirkt als mildes Desinfizienz und entzündungswidrig, darüber hinaus reagiert es schwach alkalisch.

Das erfindungsgemäße wirkstoffhaltige Pflaster kann für kosmetische sowie zur Herstellung eines Präparats für therapeutische Zwecke in der Human- und Veterinärmedizin verwendet werden.

Das rekristallisationsfreie oestrogen- und/oder gestagenhaltige Pflaster mit ausreichender Wirkstofffreisetzung enthält im Reservoir Estradiol und seine pharmazeutisch unbedenklichen Derivate allein oder in Kombination mit Gestagenen in einer Konzentration von insgesamt 1-20 Gew.%, bezogen auf die Gesamtheit der Reservoirbestandteile, und zwar in einem molaren Verhältnis von 1:1 bis 1:10.

Das estradiolhaltige Reservoir kann mindestens einen Bestandteil der Gruppe enthalten, welche Alterungsschutzmittel, Weichmacher, Antioxidantien und Absorptionsverbesserer umfaßt. Geeignete Weichmacher sind dem Fachmann bekannt und beispielsweise in der DE 37 43 946 beschrieben. Das estradiolhaltige Reservoir enthält üblicherweise Weichmacher in einem Anteil bis zu 5 Gew.%.

Weiterhin sind im wirkstoffhaltigen Reservoir auch Alterungsschutzmittel in einer Konzentration bis zu 1 Gew.% enthalten. Diese sind dem Fachmann bekannt und z.B. in der DE 37 43 946 beschrieben.

Die Materialien für die undurchlässige Rückschicht und die wiederablösbare Schutzschicht sind dem Fachmann ebenso bekannt.

Das estradiolhaltige Reservoir kann aus Lösung, aus Dispersion und auch aus der Schmelze erzeugt werden.

Das Reservoir kann aus mehreren Schichten bestehen.

Für den Fall, daß das Reservoir keine ausreichende Eigenklebrigkeit zur Haut aufweist, kann dieses mit einer zusätzlichen wirkstofffreien haftklebenden Schicht oder mit einem umlaufenden haftklebenden Rand versehen werden. Auf diese Weise ist gewährleistet, daß das transdermale Pflaster über den gesamten Applikationszeitraum auf der Haut haftet.

Ein besonders bevorzugter Aufbau des transdermalen estradiolhaltigen Pflasters ist ein Matrix-System, bei dem bekanntlich die Matrix die Steuerung für die Wirkstofffreisetzung übernimmt und dem √t-Gesetz nach Higuchi gehorcht. Das bedeutet jedoch nicht, daß nicht in besonderen Fällen auch ein Membran-System von Vorteil ist. Hierbei ist zwischen Reservoir und Haftkleberschicht eine die Wirkstofffreisetzung steuernde Membran angebracht.

Die Dicke des transdermalen Pflasters richtet sich nach den therapeutischen Erfordernissen und kann entsprechend angepaßt werden. Sie liegt üblicherweise im Bereich von 0,03-0,6 mm.

Die Erfindung wird nachfolgend anhand von Ausführungsbeispielen erläutert.

### BEISPIEL 1:

- 97,86 g: Durotak 387-2287-Lösung (50,2 g Feststoff)
- 4,0 g: Wollwachs
- 21,56 g: Ethanol und
- 10,78 g: Essigsäureethylester werden durch 2- bis 3-stündiges Rühren bei Raumtemperatur homogenisiert. Anschließend werden
- 2,0 g: Estradiol-Hemihydrat und
- 7,0 g: Norethindronacetat zugegeben und ca. 1 Stunde gerührt. Danach werden
- 16,8 g: Zinkoxid zugegeben und weitere 30 Min. gerührt. Die so erhaltene wirkstoffhaltige Klebmasse wird so auf die wiederablösbare Schutzschicht (Hostaphan RN 100, einseitig mit Silikon beschichtet - Fa. Hoechst-Diafoil) beschichtet, daß ein wirkstoffhaltiges Reservoir mit einem Flächengewicht von ca. 80 g/m² resultiert. Aus dieses Reservoir wird die undurchlässige Rückschicht (Polyesterfolie, 19 µm dick) aufkaschiert. Anschließend werden 16 cm² große Wirkstoffpflaster ausgestanzt.

### BEISPIEL 2 und 3:

Die Herstellung erfolgt wie unter Beispiel 1 beschrieben, jedoch mit in Tabelle 1 (Herstellformel) angegebenen Rohstoffmengen.

Zur Messung der Humanhautpenetration wird die Haut in die Franz-Zelle eingespannt. Auf die Haut wird ein oestrogen- und/oder gestagenhaltiges Pflaster mit einer Fläche von 1,539 cm² aufgeklebt und die Wirkstofffreisetzung bei 37 °C (Akzeptormedium: 0,9 %ige Kochsalzlösung + 0,1 % NaN₃) gemessen.

Die Prüfung auf Rekristallisationserscheinung wird visuell im Gegenlicht durchgeführt.

Die Ergebnisse sind in Tabelle 2 dargestellt.

**TABELLE 1:**

| Zusammensetzung (Angabe in g) | | | | | |
|---|---|---|---|---|---|
| Beispiel | Durotak 387-2287 Feststoff | Wollwachs | Zinkoxid | Estradiol-Hemihydrat | Norethindronacetat |
| 2 | 50,2 | 5,6 | 15,2 | 2,0 | 7,0 |
| 3 | 50,2 | 2,4 | 18,4 | 2,0 | 7,0 |

**TABELLE 2:**

| Analysenergebnisse | | | | | |
|---|---|---|---|---|---|
| Beispiel | Wirkstoffgehalt µg/16 cm² | | Humanhautpenetration µg/16 cm² (24-48 Std.) | | Rekristallisation |
| | Oes | NeA | Oes | NeA | |
| 1 | 3.200 | 11.200 | 56 | 90 | keine |
| 2 | 3.200 | 11.200 | 49 | 76 | keine |
| 3 | 3.200 | 11.200 | 48 | 79 | Keine |
| Vergleichsprodukt Evorel Conti | 3.200 | 11.200 | 33 | 46 | erheblich |
| Oes: Oesrtadiol-Hemihydrat NeA: Norethindronacetat | | | | | |

Wie die Tabelle zeigt, erhält man bei dem erfindungsgemäßen Pflaster eine deutlich bessere Penetration durch die Humanhaut gegenüber dem Vergleichsprodukt. Parallel dazu kann festgestellt werden, daß die Rekristallisation bei den erfindungsgemäßen Beispielen völlig unterbleibt.

## Patentansprüche

1. Wirkstoffhaltiges Pflaster zur kontrollierten Abgabe von Estradiol oder seinen pharmazeutisch unbedenklichen Derivaten allein oder in Kombination mit Gestagenen an die menschliche oder tierische Haut, aus einer Rückschicht, einem damit verbundenen, für die kombinierte Wirkstoffabgabe geeigneten wirkstoffhaltigen Reservoir, das unter Verwendung von Haftklebern hergestellt ist, und einer wiederablösbaren Schutzschicht, **dadurch gekennzeichnet, daß** der Haftkleber Wollwachs oder Bestandteile davon und Zinkoxid enthält.

2. Wirkstoffhaltiges Pflaster nach Anspruch 1, **dadurch gekennzeichnet, daß** das Reservoir 1-30 Gew.% Wollwachs und 1-30 Gew.% Zinkoxid enthält.

3. Wirkstoffhaltiges Pflaster nach Anspruch 1 oder 2, **dadurch gekennzeichnet, daß** das Reservoir Estradiol oder seine pharmazeutisch unbedenklichen Derivate allein oder in Kombination mit Gestagenen in einem Anteil von 1-20 Gew.%, bevorzugt 1,5-15 Gew.%, enthält.

4. Wirkstoffhaltiges Pflaster nach einem der Ansprüche 1 bis 3, **dadurch gekennzeichnet, daß** das Reservoir Estradiol oder seine pharmazeutisch unbedenklichen Derivate allein oder in Kombination mit Gestagenen in einem molaren Verhältnis von 1:1 bis 1:10 enthält.

5. Wirkstoffhaltiges Pflaster nach einem oder mehreren der Ansprüche 1 bis 4, **dadurch gekennzeichnet, daß** der Haftkleber klebrigmachende Harze in einer Konzentration von 1-50 Gew.% enthält.

6. Wirkstoffhaltiges Pflaster nach einem oder mehreren der Ansprüche 1 bis 5, **dadurch gekennzeichnet, daß** das Reservoir mindestens einen Bestandteil aus der Gruppe von Alterungsschutzmitteln, Weichmachern, Antioxidantien und Absorptionsverbesserern enthält.

7. Wirkstoffhaltiges Pflaster nach einem oder mehreren der Ansprüche 1 bis 6, **dadurch gekennzeichnet, daß** der Haftkleber ein Lösemittelhaftkleber, Dispersionshaftkleber oder Schmelzhaftkleber ist.

8. Wirkstoffhaltiges Pflaster nach einem oder mehreren der Ansprüche 1 bis 7, **dadurch gekennzeichnet, daß** die Rückschicht für die Bestandteile des Reservoirs undurchlässig ist.

9. Wirkstoffhaltiges Pflaster nach einem oder mehreren der Ansprüche 1 bis 8, **dadurch gekennzeichnet, daß** das Reservoir aus mehreren Schichten besteht und mit einer zusätzlichen wirkstofffreien haftklebenden Schicht versehen ist.

10. Wirkstoffhaltiges Pflaster nach einem oder mehreren der Ansprüche 1 bis 9, **dadurch gekennzeichnet, daß** zwischen Reservoir und Haftklebeschicht eine die Wirkstofffreisetzung steuernde Membran angebracht ist.

11. Wirkstoffhaltiges Pflaster nach einem oder mehreren der Ansprüche 1 bis 10, **dadurch gekennzeichnet, daß** das Reservoir mit einem umlaufenden haftklebenden Rand versehen ist.

12. Wirkstoffhaltiges Pflaster nach einem oder mehreren der Ansprüche 1 bis 11, **dadurch gekennzeichnet, daß** die Dicke des wirkstoffhaltigen Pflasters im Bereich von 0,03-0,6 mm liegt.

13. Nicht therapeutische Verwendung des wirkstoffhaltigen Pflasters gemäß den Ansprüchen 1 bis 12 für kosmetische Zwecke.

14. Verwendung des wirkstoffhaltigen Pflasters nach einem der Ansprüche 1 bis 12 zur Herstellung eines Präparats für therapeutische Zwecke in der Human- und Veterinärmedizin.

## Claims

1. Active substance-containing patch for controlled release of estradiol or its pharmaceutically acceptable derivatives, alone or in combination with gestagens, to the human or animal skin, consisting of a backing layer, an active substance-containing reservoir connected thereto which is suitable for combined active substance release and which has been produced using pressure-sensitive adhesives, and a detachable protective layer, **characterized in that** the pressure-sensitive adhesive contains wool wax or components thereof and zinc oxide

2. Active substance-containing patch according to Claim 1, **characterized in that** the reservoir contains 1 - 30%-wt. of wool wax and 1 - 30%-wt. of zinc oxide.

3. Active substance-containing patch according to Claim 1, **characterized in that** the reservoir contains estradiol or its pharmaceutically acceptable derivatives, alone or in combination with gestagens, in a portion of 1 - 20%-wt., preferably 1.5 - 15%-wt.

4. Active substance-containing patch according to any one of Claims 1 to 3, **characterized in that** the reservoir contains estradiol or its pharmaceutically acceptable derivatives, alone or in combination with gestagens, in a molar ratio of 1:1 to 1:10.

5. Active substance-containing patch according to one or more of Claims 1 to 4, **characterized in that** the pressure-sensitive adhesive contains tackifying resins in a concentration of 1 - 50%-wt.

6. Active substance-containing patch according to one or more of Claims 1 to 5, **characterized in that** the reservoir contains at least one component belonging to the group of age-protecting agents, antioxidants and absorption enhancers.

7. Active substance-containing patch according to one or more of Claims 1 to 6, **characterized in that** the pressure-sensitive adhesive is a pressure-sensitive solution adhesive, a pressure-sensitive dispersion adhesive or a pressure-sensitive hot-melt adhesive.

8. Active substance-containing patch according to one or more of Claims 1 to 7, **characterized in that** the backing layer is impermeable to the components of the reservoir.

9. Active substance-containing patch according to one or more of Claims 1 to 8, **characterized in that** the reservoir consists of several layers and is provided with an additional active substance-containing pressure-sensitive adhesive layer.

10. Active substance-containing patch according to one or more of Claims 1 to 9, **characterized in that** an active substance release-controlling membrane is provided between the reservoir and the pressure-sensitive adhesive layer.

11. Active substance-containing patch according to one or more of Claims 1 to 10, **characterized in that** the reservoir is provided with a circumferential, pressure-sensitive adhesive margin.

12. Active substance-containing patch according to one or more of Claims 1 to 11, **characterized in that** the thickness of the active substance-containing patch is in the range of 0.03 - 0.6 mm.

13. Non-therapeutic use of the active substance-containing patch according to Claims 1 to 12 for cosmetic purposes.

14. Use of the active substance-containing patch according to one of Claims 1 to 12 for the production of a preparation for therapeutic purposes in human or veterinary medicine.

## Revendications

1. Emplâtre contenant une substance active pour la libération contrôlée d'estradiol ou de ses dérivés pharmaceutiquement acceptables seuls ou en combinaison avec des progestatifs sur la peau humaine ou animale, constitué d'une couche dorsale, d'un réservoir contenant une substance active qui y est lié, approprié pour l'administration combinée d'une substance active, qui est produit en utilisant des adhésifs par contact, et d'une couche protectrice détachable, **caractérisé en ce que** l'adhésif par contact contient de la lanoline ou ses constituants et de l'oxyde de zinc.

2. Emplâtre contenant une substance active selon la revendication 1, **caractérisé en ce que** le réservoir contient de 1 à 30% en poids de lanoline et de 1 à 30% en poids d'oxyde de zinc.

3. Emplâtre contenant une substance active selon la revendication 1 ou 2, **caractérisé en ce que** le réservoir contient de l'estradiol ou ses dérivés pharmaceutiquement acceptables, seuls ou en combinaison avec des progestatifs en une proportion de 1 à 20% en poids, de préférence de 1,5 à 15% en poids.

4. Emplâtre contenant une substance active selon l'une des revendications 1 à 3, **caractérisé en ce que** le réservoir contient de l'estradiol ou ses dérivés pharmaceutiquement acceptables, seul(s) ou en combinaison avec des progestatifs dans un rapport molaire de 1 :1 à 1 :10.

5. Emplâtre contenant une substance active selon une ou plusieurs des revendications 1 à 4, **caractérisé en ce que** l'adhésif par contact contient des résines collantes en une concentration de 1 à 50% en poids.

6. Emplâtre contenant une substance active selon une ou plusieurs des revendications 1 à 5, **caractérisé en ce que** le réservoir contient au moins un constituant du groupe des agents de protection contre le vieillissement, des plastifiants, des antioxydants et des agents améliorant l'absorption.

7. Emplâtre contenant une substance active selon une ou plusieurs des revendications 1 à 6, **caractérisé en ce que** l'adhésif par contact est un adhésif par contact dissous dans un solvant, contenu dans une dispersion ou en masse fondue.

8. Emplâtre contenant une substance active selon une ou plusieurs des revendications 1 à 7, **caractérisé en ce que** la couche dorsale est imperméable aux constituants du réservoir.

9. Emplâtre contenant une substance active selon une ou plusieurs des revendications 1 à 8, **caractérisé en ce que** le réservoir se compose de plusieurs couches et est muni d'une couche adhésive par contact supplémentaire dépourvue de substance active.

10. Emplâtre contenant une substance active selon une ou plusieurs des revendications 1 à 9, **caractérisé en ce qu'**on introduit une membrane régulant la libération de substance active entre le réservoir et la couche adhésive par contact.

11. Emplâtre contenant une substance active selon une ou plusieurs des revendications 1 à 10, **caractérisé en ce que** le réservoir est muni d'une bordure périphérique adhérant par contact.

12. Emplâtre contenant une substance active selon une ou plusieurs des revendications 1 à 11, **caractérisé en ce que** l'épaisseur de l'emplâtre contenant une substance active se situe dans un intervalle allant de 0,03 à 0,6 mm.

13. Utilisation non thérapeutique de l'emplâtre contenant une substance active selon les revendications 1 à 12 dans des buts cosmétiques.

14. Utilisation de l'emplâtre contenant une substance active selon l'une des revendications 1 à 12 pour la production d'une préparation dans des buts thérapeutiques dans la médecine humaine et vétérinaire.
